(19) Europäisches Patentamt
European Patent Office
Office européen des brevets

(11) Veröffentlichungsnummer: **0 031 926**
A2

(12) # EUROPÄISCHE PATENTANMELDUNG

(21) Anmeldenummer: 80107881.7

(22) Anmeldetag: 13.12.80

(51) Int. Cl.$^3$: G 01 N 33/74
G 01 N 33/56

(30) Priorität: 28.12.79 DE 2952635

(43) Veröffentlichungstag der Anmeldung:
15.07.81 Patentblatt 81/28

(84) Benannte Vertragsstaaten:
BE DE FR IT

(71) Anmelder: HOECHST AKTIENGESELLSCHAFT
Postfach 80 03 20
D-6230 Frankfurt/Main 80(DE)

(72) Erfinder: Allen, Robert Manning
11 Congreve Tinkers Bridge
Milton Keynes Buckinghamshire(GB)

(72) Erfinder: Redshaw, Martin Ralphs
20 Medale Road Beanhill
Milton Keynes Buckinghamshire(GB)

(54) **Radioimmunobesteck zur Bestimmung von Progesteron, Verfahren zur radioimmunologischen Progesteronbestimmung und Verwendung des Radioimmunbesteckes.**

(57) Radioimmunobesteck zur Bestimmung von Progesteron, dadurch gekennzeichnet, daß es als Reagenzien

a) ein Progesteronderivat, das in 3-Stellung über eine Brücke mit einem Rest substituiert ist, der ein oder mehrere Atome eines Radiojodisotopes trägt;
b) gegen ein Propesteron/Proteinkonjugat erzeugte Antikörper, wobei das Protein über eine Brückengruppe in 11-Stellung an das Progesteron gebunden ist;
c) Progesteronstandards und
d) ein Trennmittel enthält,

Verfahren zur radioimmunologischen Bestimmung von Progesteron und Verwendung des Radioimmunobesteckes zur Progesteronbestimmung im Serum oder in der Milch.

EP 0 031 926 A2

HOECHST AKTIENGESELLSCHAFT HOE 77/F 280 Dr.MD/cr

Radioimmunobesteck zur Bestimmung von Progesteron, Verfahren zur radioimmunologischen Progesteronbestimmung und Verwendung des Radioimmunbesteckes

Gegenstand der Erfindung ist ein Radioimmunbesteck zur Bestimmung von Progesteron, ein Verfahren zur radioimmunologischen Progesteronbestimmung und die Verwendung dieses Besteckes.

Bei nicht schwangeren Frauen vor dem Klimakterium wird Progesteron hauptsächlich vom Corpus luteum des Ovariums, in geringer Menge auch von den Nebennieren synthetisiert, wo es als Vorstufe für die Synthese anderer Steroide dient. Bei Männern werden geringe Mengen von den Nebennieren und von den Hoden synthetisiert.

Bei normalen Männern und bei Frauen nach dem Klimakterium liegt der Serumprogesteronspiegel im allgemeinen bei weniger als etwa 2 ng/ml (Bereich von 0,4 bis 4,4 ng/ml). Bei der normalen ovulierenden Frau ändert sich der Spiegel jedoch entsprechend der Phase des Geschlechtszyklus. Während der follikulären Phase (Tage 1 bis 12) des Zyklus liegt der Serumprogesteronspiegel im Bereich 0,4 bis 4,4 ng/ml. Die Ovulation findet etwa am 14. Tag statt (Zyklusmitte).

Während sich das Corpus luteum aus dem gesprungenen Follikel entwickelt, synthetisiert es steigende Mengen Progesteron aus dem Cholesterinvorrat, wobei der Serumprogesteronspiegel 7 bis 10 Tage nach der Ovulation (Tage 21 bis 24 des Zyklus) ein zwischen etwa 2 und 20 ng/ml variierendes Maximum erreicht. Progesteron ist fiebertreibend und erhöht die Körpertemperatur um etwa 0,5°C in Zyklusmitte. Die Hauptfunktionen des Progesterons bestehen darin, das Endometrium für die

Implantation vorzubereiten und, falls Befruchtung eintritt, die Schwangerschaft aufrechtzuerhalten. Bei fehlender Befruchtung bleibt das Corpus luteum nicht bestehen, und der Progesteronspiegel fällt rasch auf das Grundniveau gegen Ende des Zyklus (Tag 28), wenn die Menstruation erneut beginnt. Der erhöhte Serumspiegel des Progesterons während des Zyklus wird von parallelen Zunahmen des Progesteronstoffwechselprodukts Pregnandiol im Urin begleitet.

Tritt Befruchtung ein, so beginnt der Serumprogesteronspiegel am Tag des Maximums des luteinisierenden Hormons anzusteigen und erreicht im Verlauf der folgenden 7 Tage ein Plateau bei etwa 10 bis 35 ng/ml. Bis etwa zur 10. Woche schwankt der Spiegel üblicherweise zwischen den Grenzen dieses Plateaus, während die Placenta die Progesteronerzeugung übernimmt. Danach erfolgt ein stetiger Anstieg in Serumprogesteron bis zu einem Spiegel von etwa 100 bis 300 ng/ml kurz vor dem Ende der Schwangerschaftszeit, zu welcher Zeit die Serumkonzentration zu fallen beginnt und am Ende der Schwangerschaftszeit etwa 70 % bis 80 % ihres Maximumspiegels erreicht.

Die Bestimmung des Serumprogesteronspiegels ist besonders nützlich bei Unfruchtbarkeitsstudien. Eine Einzelmessung oder Reihenmessungen können der Feststellung dienen, ob Ovulation stattgefunden hat, insbesondere nach einer Behandlung. Diese Behandlung kann eine Therapie mit menschlichem klimakterischen Gonadotropin, Clomiphenstimulierung oder neuerdings die Auslösung einer Hormonstimulierung der Gonadotropinsynthese und -absonderung in der Hypophyse einschließen. Eine natürliche Bestätigung erhält man durch erfolgreiche Empfängnis.

Ein ähnliches Progesteronspiegelschema beobachtet man

während des Reproduktionszyklus bei anderen Säugetieren, obwohl die relative Bedeutung der Placenta und des Corpus luteum bei der Progesteronerzeugung von Säugetier zu Säugetier verschieden ist. Die Reproduktionsleistung ist bei der Tierhaltung wichtig, da ein Produktionsverlust wegen mangelnder Identifizierung von nichtträchtigen Tieren kostspielig sein kann. Die Diagnose der Trächtigkeit bei Kühen ist von besonderer Wichtigkeit, aber es ist schwierig oder undurchführbar, diese Diagnose im Frühstadium durch klinische Diagnose oder chemische Analyse von Urinöstrogenen, Plasmagonadotropinen oder Plasmaprogesteron genau zu stellen. Kürzlich wurde jedoch eine ausgezeichnete Korrelation zwischen den Plasma- und Milchprogesteronspiegeln gefunden, wobei der letztere erheblich höher liegt.

Die bisher vorgeschlagenen radioimmunologischen Bestimmungssysteme zur Bestimmung des Progesteronspiegels weisen einen erheblichen Nachteil auf, da eine vorangehende Reinigungsstufe erforderlich ist.Ferner verwendete man bis vor kurzem tritiumhaltige Indikatoren mit den damit verbundenen Problemen bei der ß-Flüssigkeitsscintillationszählung.Zu diesen Nachteilen gehören verhältnismäßig unempfindliche Bestimmungen, langwierige und zeitraubende Arbeitsweisen und die eigentlichen Probleme der ß-Scintillationszählung: die Probenahme aus der überstehenden Lösung, Löschung der Radioaktivität, der Preis der Reagenzien und deren anschließende Beseitigung. Die Einführung von $\gamma$-Strahlen aussendenden Isotopen als Indikatoren hat die Zählung vereinfacht, aber sogar Bestimmungen unter Verwendung solcher Indikatoren erforderten immer noch eine Vorbehandlung der Serumproben. Die angewandte Vorbehandlung ist zum Beispiel wie folgt:

SERUMRPOBE

↓

Petroläther zusetzen

↓

15 Minuten schütteln

↓

wässrige Schicht einfrieren

↓

Petroläther abgiessen

↓

zur Trockne eindampfen

↓

Puffer zusetzen

↓

umwirbeln und 30 Minuten stehen lassen

↓

BESTIMMUNG

Weitere Stufen, beispielsweise Gewinnungsversuche und Chromatographie, werden häufig bei der Vorbehandlung eingeschlossen.

Es besteht die Möglichkeit, anstelle des tritiumhaltigen Indikators ein radioaktives Jodisotop als $\gamma$-Strahler bei der radioimmunologischen Progesteronbestimmung einzusetzen. Bei den meisten radioimmunologischen Bestimmungsmethoden unter Verwendung jodierter Indikatoren setzt man homologe Systeme ein, d.h. sowohl der Indikator als auch das zur Bildung des Konjugats verwendete Protein sind in derselben Stellung an das Steroid gebunden und benutzen dieselbe Brückengruppe, zum Beispiel 11$\alpha$-Bernsteinsäurehalbester-TME-indikator und 11$\alpha$-Bernsteinsäurehalbester-proteinkonjugat (TME-Tyrosinmethylester). Derartige Bestimmungsmethoden sind jedoch verhältnismäßig unempfindlich, und es wurde vorgeschlagen, zur Verbesserung der Empfindlichkeit heterologe Systeme zu verwenden. In einem solchen System

sind die Brückengruppen im Indikator und Konjugat unterschiedlich, und/oder das konjugierende Protein und der Indikator sind an verschiedenen Stellen an das Steroid gebunden. Scarisbrick und Cameron, im Journal of Steroid Biochemistry 1975, Band 6, S. 51-55, vergleichen die relativen Empfindlichkeiten homologer und heterologer Bestimmungssysteme, jedoch erfordern die Bestimmungssysteme in allen Fällen die zeitraubende und umständliche Probenvorbehandlungsstufe, wie oben beschrieben, vor der Einführung in die eigentliche Bestimmung.

Der vorliegenden Erfindung liegt die überraschende Beobachtung zugrunde, daß die Verwendung bestimmter heterologer Systeme es ermöglicht, hinreichende Empfindlichkeit zu erzielen, so daß man eine Progesteronbestimmung direkt an einer Probe einer biologischen Flüssigkeit, zum Beispiel Serum, Plasma, Urin oder Milch, durchführen kann.

Die vorliegende Erfindung stellt ein Radioimmunobesteck und eine Bestimmungsmethode für Progesteron zur Verfügung, welche dadurch gekennzeichnet ist, daß man in saurem Medium, vorzugsweise einem sauren Puffer, eine Flüssigkeitsprobe mit einer bekannten Menge von gegen ein Progesteron/Proteinkonjugat erzeugten Antikörpern, wobei das Protein über eine Brückengruppe in 11-Stellung, im allgemeinen $11\alpha$-Stellung, an das Progesteron gebunden ist, und mit einer bekannten Menge eines Progesteronderivats, das in 3-Stellung über eine Brücke mit einem Rest substituiert ist, welcher ein oder mehrere Atome eines Jodradioisotops trägt, inkubiert, das in dem entstandenen Antikörper /Antigenkomplex gebundene Steroid von der freien Steroidkomponente abtrennt und deren Radioaktivität oder die des Antikörper/Antigenkomplexes mißt. Im allgemeinen wird das

Antiserum zuletzt zugesetzt, und der Indikator wird vorzugsweise zum Standard bzw. zur Probe gegeben.

Die Flüssigkeit, in der die Bestimmung auszuführen ist, ist insbesondere eine biologische Flüssigkeit oder ein wässriger Extrakt eines biologischen Gewebes oder Organs, beispielsweise ist dies Serum,Plasma, Urin oder Milch.

Die Bestimmungsmethode selbst ist eine übliche radioimmunologischeBestimmung, und die Verwendung eines ein Antiserum gegen ein Progesteron-11α-proteinkonjugat und einen 3-jodierten Indikator umfassenden heterologen Systems bei einer solchen Bestimmung wurde bereits von Scarisbrick und Cameron (a.a.O.) vorgeschlagen, jedoch ist, wie oben herausgestellt, bei ihrer Bestimmung eine Vorbehandlung, bei der es sich um eine Petrolätherextraktion wie oben beschrieben handelt, eine wesentliche Stufe. Es ist überraschend, daß diese bisher für sämtliche radioimmunologischen Progesteronbestimmungsmethoden als unumgänglich erachtete Stufe weggelassen werden kann und daß man die Bestimmung direkt an der Probe durchführen kann. Dies führt zu erheblichen Zeiteinsparungen bei der Durchführung dieser Bestimmung, wobei die Ergebnisse der Bestimmung, zusammen mit einem großen Durchsatz unbekannter Proben, auch schneller zur Verfügung stehen können. Bei Bestimmungssystemen, welche die Vorextraktionsstufe erfordern, ist dies unmöglich.

Wie oben erwähnt ist die eigentliche Bestimmung eine herkömmliche radioimmunologische Bestimmung mit Bindungskonkurrenz, d.h. sie ist abhängig von der Konkurrenz zwischen unmarkiertem Progesteron und einem radiojodierten Progesteronderivat um die Bindungsstellen eines progesteronspezifischen Antikörpers. Der an den Antikörper gebundene Anteil an jodiertem Progesteron

ist der Menge des vorhandenen unmarkierten Progesterons umgekehrt proportional. Dieser Anteil wird dadurch gemessen, daß man die gebundenen und freien Komponenten trennt, im allgemeinen durch Adsorption der letzteren, und die Radioaktivität der einen oder der anderen mißt.

Die erfindungsgemäße radioimmunologische Bestimmungsmethode erfordert als ein Reagenz ein Antiserum gegen Progesteron-11-proteinkonjugat, im allgemeinen ein $11\alpha$-Proteinkonjugat, und als weiteres Reagenz einen 3-jodierten Progesteronindikator. Entscheidend sind die Bindungsstellen des Proteins und der jodierten Gruppe an dem Progesteron, während die Art des Proteins, der jodierten Gruppe und der Brückengruppen von geringerer Bedeutung sind.

Die theoretische Grundlage für die Bildung von Protein/Steroidkonjugaten ist wohlbekannt, und mehrere Methoden zur Bildung solcher Konjugate stehen zur Verfügung. Infragekommende Proteine sind beispielsweise Ovalbumin (OA), Rinderserumalbumin (BSA), menschliches Serumalbumin (HSA), Hämocyanin aus Schlüssellochnapfschnecken [Fissurella] (KLH), Rindergammaglobulin und Polylysin.

Allgemein entsprechen die nach bekannten Verfahren einzuführenden Brückengruppen der allgemeinen Formel $11\alpha$-O-R- worin R für

$$-\overset{O}{\overset{\|}{C}}-$$

$$-\overset{O}{\overset{\|}{C}}-(CH_2)_n-\overset{O}{\overset{\|}{C}}-$$

wobei n eine ganze Zahl von 1 bis 6 ist

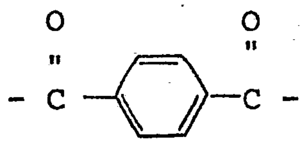

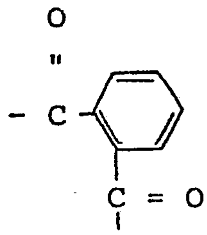

steht.

Unter diesen Systemen wird durch eine Bernsteinsäurehalbesterbrücke an das Steroid gebundenes Ovalbumin bevorzugt.

Das Protein/Steroidkonjugat wird dazu verwendet, ein Antiserum mit hoher Affinität für Progesteron, aber einem begrenzten Ausmaß an Unspezifität im Bereich der 3-Stellung zur Verwendung in der erfindungsgemäßen Bestimmung zu erzeugen. Das Antiserum kann in irgendeinem Säugetier, beispielsweise einem Pferd, einer Ziege oder einem Schaf, gebildet werden, aber Kaninchen und Meerschweinchen werden bevorzugt. Methoden zur Erzeugung eines Antiserums in einem Wirtstier sind ebenfalls wohlbekannt. Im vorliegenden Fall mit Kaninchen wendet man vorzugsweise die intradermale Vielstellenmethode an, wobei die Zubereitungen zur Injektion neben dem Antigen ein wässriges Immunogen oder eine Hilfsstoffemulsion enthalten, gegebenenfalls mit Nachinjektionen nach Beurteilung der Reaktion.

Wie bei dem Proteinkonjugat ist bei dem jodierten Indikator die Lage der Bindung an das Steroidmolekül und in geringerem Grade die Art der Gruppe selbst und der Brückengruppe wichtig. Es kommt jegliche jodierbare Gruppe infrage, vorzugsweise eine jodierbare aromatische Gruppe oder aromatisch-heterocyclische Reste, beispielsweise Tyrosinmethylester, Tyraminderivate oder insbesondere Histamin. Das allgemein verwendete Jodradioisotop ist $^{125}J$, doch ist $^{131}J$ ebenfalls brauchbar. Zur Jodierung wird in bekannter Weise ein Alkalijodid in Gegenwart von Oxidationsmitteln verwendet.

Die Art der in bekannter Weise eingeführten Brückengruppe zwischen der jodierbaren Gruppe und dem Steroid ist wiederum nicht von großer Bedeutung. Diese Brückengruppe kann dieselbe sein wie die für das Konjugat eingesetzte, aber vorzugsweise ist sie verschieden. Bevorzugte Brückengruppen sind (O-Carboxyalkyl)-oximgruppen der allgemeinen Formel

$$-NO-(CH_2)_n-\overset{O}{\overset{\|}{C}}-,$$

worin n eine ganze Zahl von 1 bis 6 ist. Darunter hat sich die Carboxymethyloximgruppe besonders bewährt.

Die bevorzugte Reagenzienkombination für die erfindungsgemäße Bestimmungsmethode besteht aus einem gegen Progesteron-11α-bernsteinsäurehalbester-ovalbumin erzeugten Antiserum und Progesteron-3-(O-carboxymethyl)-oxim-[$^{125}J$]-jodhistamin.

Steroidhormone finden sich häufig in Körperflüssigkeiten lose an steroidbindende Proteine gebunden, durch die sie im Blut transportiert werden. Zur quantitativen Bestimmung ist eine Verdrängung des Hormons erforderlich. Dies trifft zu, gleich ob die Bestimmungsmethode direkt ist oder nicht. Die Verdrängung läßt sich durch Zusatz eines nicht kreuzreagierenden Steroids im Überschuß, durch Hitzedenaturierung des bindenden Proteins, durch Verwendung eines sauren Puffers oder durch eine andere bekannte Methode bewirken. Die erfindungsgemäße Bestimmungsmethode wird in einem sauren Medium durchgeführt, im allgemeinen einem sauren Puffer, der das Progesteron aus irgendeinem bindenden Protein freisetzt, wo die Bindungsaffinität an jenes

Protein verhältnismäßig niedrig ist. Der Puffer verursacht jedoch keine Freisetzung von Progesteron aus dem erfindungsgemäß verwendeten Antikörper, da die Bindungsaffinität verhältnismäßig hoch ist. Der empfohlene Puffer ist Phosphat-Citrat mit einem pH von 3,5 ± 0,5. Vorzugsweise liegt jedes Reagenz in dem sauren Puffer vor.

Die Bestimmung wird auf herkömmliche Weise durchgeführt, beispielsweise erstellt man einen Satz Röhrchen, die jeweils ein Aliquot an 3-jodiertem Indikator und entweder eine bekannte Menge einer Standardprogesteronlösung in einem geeigneten, von Steroid befreiten Verdünnungsmittel oder eine unbekannte Probe enthalten. Vorzugsweise gibt man den Indikator zum Standard oder zur Probe. Ein Aliquot des Antiserums wird in jedes Röhrchen gegeben, dessen Inhalt gut vermischt und stehengelassen wird, vorzugsweise bei 0 - 4°C. Die Bestimmung läßt sich jedoch auch bei höheren Temperaturen, z.B. Raumtemperatur oder 37°C, durchführen. Nach Ablauf einer gewissen Zeit, vorzugsweise 21/2 - 5 Stunden, wird jedem Röhrchen ein Aliquot eines Trennmittels zugesetzt. Das Trennmittel ist beispielsweise ein zweiter Antikörper oder $(NH_4)_2SO_4$, jedoch vorzugsweise Aktivkohle. Man läßt dem Trennmittel genügend Zeit, um das freie Steroid zu binden, trennt es vom Rest des Röhrcheninhalts, im Falle von Aktivkohle vorzugsweise durch Zentrifugieren, und bestimmt die Radioaktivität der festen oder flüssigen Phase. Im Falle von Aktivkohle als Adsorptionsmittel wird vorzugsweise die feste Phase, d.h. die Radioaktivität des freien (nichtantikörpergebundenen) jodierten Indikators gemessen.Andernfalls kann man eine Probe der überstehenden flüssigen Phase nehmen und zählen. Man erstellt eine Standardkurve, und die in der Probe vorliegende

Menge Progesteron kann aus der Kurve interpoliert werden.

Im Fall von Serum- oder Plasmaproben führt man die Bestimmung vorzugsweise wie folgt durch:

Die Standards werden so hergestellt, daß sie nominell bis zu 16 ng Progesteron pro ml enthalten, zweckmäßigerweise 0, 1, 2, 4, 8 bzw. 16 ng/ml. Die Empfindlichkeit der Bestimmungsmethode ist so hoch, daß bei Verwendung der Reagenzienkombination und der obigen Standards nur 20 µl einer Plasmaprobe erforderlich sind. Sämtliche Reaktionsschritte werden vorzugsweise bei 0 bis 4°C durchgeführt. Nach Zusatz des Tracers und des Antiserums läßt man die Röhrchen 1-5 Stunden stehen. Nach Zusatz des Aktivkohleadsorptionsmittels läßt man die Röhrchen 5 - 15 Minuten stehen, vorzugsweise bei 0 bis 4°C und zentrifugiert dann 15 Minuten lang bei 750 bis 2000 g. Die überstehende flüssige Phase wird abgegossen und die Aktivkohle 60 Sekunden lang gemessen.

Unter Verwendung der obigen Standards kann man Progesteron im Bereich von 1 bis 16 ng/ml genau bestimmen. Höhere Konzentrationen, beispielsweise bis zu etwa 150 ng/ml, wie sie sich während der Schwangerschaft finden, können durch vorherige Verdünnung der Probe mit einem Serum, das eine vernachlässigbare Menge Progesteron enthält, zum Beispiel männliches Pferdeserum, genau bestimmt werden.

Die Empfindlichkeit der Bestimmungsmethode kann erhöht werden, d.h. ein Spiegel unter etwa 1 ng/ml kann durch entsprechende Verdünnung des Antiserums, durch die Verwendung geeigneter Standards und/oder durch Verlängerung der Inkubationszeit vor der Trennung der freien und gebundenen Steroidkomponenten genau bestimmt werden. Auf diese Weise läßt sich der Progesteronspiegel

während der Follikelphase bei Frauen und der Progesteronspiegel bei erwachsenen Männern feststellen.

Wie oben angegeben kann die erfindungsgemäße Bestimmungsmethode an Serum- oder Plasmaproben von Männern oder von Frauen nach oder vor der Menopause durchgeführt werden. Bei Frauen kann die Bestimmungsmethode zur Feststellung der Schwangerschaft angewandt werden. Wenn in Verbindung mit einer hCG-Bestimmung während der Schwangerschaft im Fall einer drohenden Fehlgeburt durchgeführt, besteht die Möglichkeit, die Ursache der drohenden Fehlgebut entweder einem geschädigten Fötus oder einen Progesteronmangel zuzuordnen.

Die Bestimmung ist bei der Untersuchung der Infertilität besonders nützlich, sofern diese durch Fehlen der Ovulation verursacht ist, sowie bei der Verfolgung der Wirkung von Arzneimitteln, die zur Behandlung eines solchen Zustands verabreicht wurden. Eine von einer Frau, die natürlich oder unter dem Einfluß von Arzneimitteln ovuliert, erhaltene Plasmaprobenreihe zeigt den oben beschriebenen Anstieg und Abfall im Progesteronspiegel. Bei fehlender Ovulation beobachtet man keinen Anstieg des Progesteronspiegels in der Lutealphase. Wir haben gefunden, daß bei Verwendung der erfindungsgemäßen Bestimmungsmethode ein Progesteronspiegel von 5 ng/ml Ovulation und ein Spiegel von 12 ng/ml oder höher ausreichende Lutealfunktion anzeigt. Es ist jedoch nicht nötig, über den ganzen Geschlechtszyklus Proben zu nehmen: 5 bis 9 Tage nach der Ovulation oder nach der Verabreichung eines ovulationsstimulierenden Arzneimittels ist im allgemeinen zweckmäßig, und 7 Tage danach scheint für die Entnahme einer Einzelprobe optimal zu sein.

Wie oben erwähnt haben wir gefunden, daß die erfindungsgemäße radioimmunologische Methode zur Bestimmung des Progesteronspiegels in Milch vereinfacht. Diese Methode, die ebenfalls zur Erfindung gehört, ist bei der Tierhaltung nützlich und erlaubt es, leicht und zu einem frühen Stadium festzustellen, ob ein Tier trächtig ist oder nicht, und ferner die Ovarialfunktion zu verfolgen. Die Bestimmung kann an der Milch irgendeines betriebsmäßig gehaltenen Tieres, beispielsweise einer Stute, eines Mutterschweins, einer Ratte oder insbesondere einer Kuh, durchgeführt werden.

Die Bestimmung an Milch wird wie oben beschrieben durchgeführt. Die verwendeten Standards werden so hergestellt, daß sie entsprechende Mengen Progesteron enthalten. Im Fall von Kühen beläuft sich dies nominell bis auf vorzugsweise 32 ng Progesteron pro ml, vorteilhafterweise nominell 0, 2, 4, 8, 16 bzw. 32 ng/ml. Das an Steroid verarmte Verdünnungsmittel für die Standards ist eine Milchzubereitung, die weitgehend progesteronfrei ist, zum Beispiel eine aus Magermilch, insbesondere fettarmem, handelsüblichen Magermilchpulver hergestellte Zubereitung oder Milch von Kühen, die in der Brunst stehen.
Gegenstand der Erfindung ist dementsprechend weiterhin die Verwendung des erfindungsgemäßen Radioimmunoassays zur Feststellung der Schwangerschaft und/oder Ovarialfunktion,welche dadurch gekennzeichnet ist, daß man an einer von einem weiblichen Säugetier während einem oder mehreren Geschlechts- oder Reproduktionszyklen erhaltenen Probe bzw. Probenreihe die erfindungsgemäße Bestimmung durchführt. Im Fall von betriebsmäßig gehaltenen Tieren werden vorzugsweise Milchproben analysiert, jedoch verwendet man im Fall von Menschen im allgemeinen Plasma- oder Serumproben.
Zur Feststellung der Ovulation im letzteren Fall sollte,

wenn man nur eine Probe nehmen will, diese in der vermutlichen Lutealphase, vorzugsweise 5 bis 9 Tage nach der vermutlichen Ovulation, insbesondere 7 Tage danach, gezogen werden.

Gegenstand der Erfindung ist ferner ein Radioimmunobesteck zur Bestimmung von Progesteron, das als Reagenzien

a) ein Progesteronderivat, das in 3-Stellung über eine Brücke mit einem Rest substituiert ist, der ein oder mehrere Atome eines Radiojodisotopes, vorzugsweise J-125 trägt;
b) ein gegen ein Progesteron-11-proteinkonjugat, vorzugsweise ein 11 -Proteinkonjugat, erzeugtes Antiserum, wobei das Protein über eine Brückengruppe in 11-Stellung an das Progesteron gebunden ist;
c) Progesteronstandards, die vorzugsweise nominell 0,1, 2, 4, 8 bzw. 16 ng/ml im Fall von Serum- und Plasmabestimmungen sowie 0, 2, 4, 8, 16 bzw. 32 ng/ml im Fall von Kuhmilchbestimmungen enthalten, sowie
d) ein Trennmittel, vorzugsweise ein Aktivkohleadsorbtionsmittel, vorzugsweise zusammen mit Anweisungen zur Durchführung der Bestimmung enthält.

Als weiterer Bestandteil des Bestecks kann eine Testprobe, die eine bekannte Menge Progesteron im entsprechenden Verdünnungsmittel, z.B. Serum oder Milch enthält, vorgesehen sein.

Mindestens eines der Reagenzien und vorzugsweise sämtliche Reagenzien liegen in einem sauren Medium vorzugsweise einem sauren Puffer, vor.

Die verschiedenen Reagenzien werden vorzugsweise gefriergetrocknet und haben vorzugsweise die oben im Bezug auf die Bestimmungsmethode beschriebene Beschaffenheit.

Die Kreuzreaktivität verschiedener Steroide bei der erfindungsgemäßen Bestimmungsmethode ist unten angegeben.

Tabelle

| Steroid | % Kreuzreaktivität |
|---|---|
| Progesteron | 100 |
| Pregnenolon | 10,5 |
| Pregnenolonsulfat | 0,4 |
| Pregnenolonglucuronid | 10,0 |
| 5α-Pregnan-3,20-dion | 6,0 |
| 5ß-Pregnan-3α, 20α-diol | 0,7 |
| Desoxycorticosteron | 0,01 |
| Corticosteron | 2,7 |
| 20α-Hydroxyprogesteron | 0,25 |
| 5ß-Pregnan-3α-ol-20-on | 8,0 |
| 17α-Hydroxyprogesteron | 1,3 |

Sonstige allgemein vorkommende Steroide und synthetische Progestine zeigen vernachlässigbare Kreuzreaktion bei der Bestimmung. Die obige Tabelle zeigt, daß Pregnenolon und Pregnenolon-3-derivate eine gewisse Kreuzreaktivität aufweisen. Dies ist nötig, damit das erfindungsgemäße heterologe System auf die Untersuchung des zirkulierenden Serumprogesterons und des Progesteronspiegels in Milch angewandt werden kann.

Der unter Verwendung der erfindungsgemäßen Methode bestimmte Progesteronspiegel in menschlichem Serum oder Plasma liegt unweigerlich höher als der bei Verwendung herkömmlicher Bestimmungsmethoden erhaltene Spiegel, wo eine Vorextraktion erforderlich ist, denn zum Teil tritt ein gewisser Arbeitsverlust, im allgemeinen 10-20 %, an Steroid während der Vorbehandlung in den herkömmlichen Bestimmungsmethoden auf. Ferner führt die Gegenwart verhältnismäßig hoher Konzentrationen an Pregnenolon-konjugaten und sonstigen

störenden Substanzen zu leicht erhöhten Werten bei der vorliegenden Bestimmungsmethode gegenüber den Ergebnissen einer Bestimmungsmethode, die eine vorherige Extraktion mit einem organischen Lösungsmittel einschließt, wobei die Konjugate in der wässrigen Phase verbleiben. Natürlich kann man die zu untersuchende Flüssigkeitsprobe auch vor der radioimmunologischen Bestimmung extrahieren. Dies ermöglicht einen direkten Vergleich mit den bei Verwendung von radioimmunologischen Bestimmungen, welche die Extraktionsstufe erfordern, erhaltenen Ergebnissen, ist aber als allgemeine Arbeitsweise nicht notwendig, da die mit der erfindungsgemäßen radioimmunologischen Bestimmung erhaltenen Ergebnisse in sich widerspruchsfrei und natürlich sehr viel schneller als gegenwärtig möglich zu erhalten sind.

Beispiel 1

Bestimmung von Progesteron im Serum

Ein Besteck zur radioimmunologischen Progesteronbestimmung im Serum besteht aus den folgenden Reagenzien:

1. Mit $^{125}J$ jodiertes Progesteron, das am Herstellungsdatum nicht mehr als 5 µCi $^{125}J$ enthält, in Puffer gefriergetrocknet (1 Fläschchen).

2. Kaninchen-Antiprogesteronserum, in Puffer gefriergetrocknet (1 Fläschchen).

3. Progesteron-Referenzstandards, die nominell 0, 1, 2, 4, 8 bzw. 16 ng Progesteron /ml enthalten, in Pferdeserum gefriergetrocknet (6 Fläschchen).

4. Testersum, gefriergetrocknetes menschliches Serum (1 Fläschchen).

5. Aktivkohletrennreagenz (1 Fläschchen).

6. Koordinatenpapier/Merkblätter.

Arbeitsanleitung:

Die im Besteck enthaltenen Reagenzien werden in destilliertem Wasser gelöst bzw. suspendiert. Schaumbildung ist zu vermeiden. Zur Rekonstitution werden folgende Wassermengen benötigt:

| Reagenz | Zugabe von dest. $H_2O$ |
|---|---|
| Standards $S_0$-$S_5$ | 500 µl |
| Testserum | 500 µl |
| Progesteron- $^{125}J$ | 20 ml |
| Anti-Progesteron-Serum | 20 ml |
| Aktivkohle-Reagenz | 50 ml |

Die Standard- und Testerum-Fläschen werden kurz aufgestoßen, um am Stopfen haftende Partikel abzulösen. Dann erst werden die Abreißkappen und Gummistopfen vorsichtig entfernt und letztere umgekehrt auf die Arbeitsplatte gelegt. Nach Zugabe von genau 500 µl destilliertem Wasser (geeignete Mikropipette) setzt man die entsprechenden Stopfen wieder auf und wartet bis zum vollständigen Lösen der Lyophilisate (ca. 10 Min.). Durch kreisende Bewegungen und/oder Rollen der Fläschchen zwischen den Händen wird der Lösungsvorgang unterstützt und die erhaltene Lösung gemischt.

Das Progesteron-$^{125}$J und das Anti-Progesteron-Serum sind einfach und schnell in 20 ml destilliertem Wasser zu lösen. Durch kreisende Bewegung wird die Lösung gemischt.

Das Aktivkohle-Reagenz wird in ein 100 ml-Becherglas gegeben und nach Zugabe von 50 ml dest. Wasser leicht gerührt, um eine homogene Suspension zu erhalten (geeignet ist die Benutzung eines Magnetrührers). Die Suspension muß später während des Dispensierungsvorganges erhalten bleiben, fortgesetztes Rühren ist also erforderlich.

Progesteron-$^{125}$J, Anti-Progesteron-Serum und Aktivkohle-Suspension müssen vor Beginn des Assays of 0-4°C gekühlt werden. Die Verwendung von Eis-Wasser-Bädern wird empfohlen.

Durchführung der Bestimmung

1. Eine genügende Anzahl von Inkubationsröhrchen (3-5 ml) werden, wie in der nachfolgenden Tabelle 1 angegeben, numiert (Gesamtaktivität, 6 Standards, Testserum und die Anzahl Serumproben). In jedem Fall wird der Ansatz von Doppelproben empfohlen. Die Röhrchen werden in geeignete Reagenzgestelle gesetzt und in Eiswasser gestellt oder in anderer Weise auf

0-4°C gekühlt.

2. Die klar gelösten Standards werden nochmals auf dem Mischrotor kurz gemixt und in 20 µl Aliquots in die dafür vorbereiteten Röhrchen (Nr. 3-14) dispensiert. Zu empfehlen ist, für jeden Standard eine neue Pipettenspitze zu nehmen.

3. 20 µl des klar gelösten und auf dem Mischrotor nochmals gemixten Testserums werden in die Reagenzröhrchen 15 und 16 pipettiert, dann die Patientenserum-Proben sorgfältig gemixt und je 20 µl in die dafür vorbereiteten Tuben 17, 18 etc. pipettiert. Für jede Patientenprobe ist eine neue Pipettenspitze einzusetzen.

   Beim Pipettieren von 20 µl ist größte Sorgfalt erforderlich.

4. 200 µl des auf 0-4°C gekühlten Progesteron-$^{125}$J-Tracers werden in jedes der vorbereiteten Röhrchen pipettiert.

5. Alle Reagenzröhrchen werden sorgfältig auf dem Mischrotor gemixt.

Tabelle 1

| | | Gesamtaktivität | | Standards (µl) | | | | | | | | | | | | Testserum (µl) | | Serumproben (µl) | | | |
|---|---|---|---|---|---|---|---|---|---|---|---|---|---|---|---|---|---|---|---|---|---|
| Nr. der Teströhrchen | | 1 | 2 | 3 | 4 | 5 | 6 | 7 | 8 | 9 | 10 | 11 | 12 | 13 | 14 | 15 | 16 | 17 | 18 | 19 | 20 |
| Standards | Gehalt nominell | | | | | | | | | | | | | | | | | | | | |
| $S_0$ | 0 ng/ml | | | 20 | 20 | | | | | | | | | | | | | | | | |
| $S_1$ | 1 ng/ml | | | | | 20 | 20 | | | | | | | | | | | | | | |
| $S_2$ | 2 ng/ml | | | | | | | 20 | 20 | | | | | | | | | | | | |
| $S_3$ | 4 ng/ml | | | | | | | | | 20 | 20 | | | | | | | | | | |
| $S_4$ | 8 ng/ml | | | | | | | | | | | 20 | 20 | | | | | | | | |
| $S_5$ | 16 ng/ml | | | | | | | | | | | | | 20 | 20 | | | | | | |
| Testerum | Gehalt deklariert | | | | | | | | | | | | | | | 20 | 20 | | | | |
| Serumprobe | | | | | | | | | | | | | | | | | | 20 | 20 | | |
| Serumprobe etc. | | | | | | | | | | | | | | | | | | | | 20 | 20 |
| Progesteron-$^{125}J$ (0-4°C) | | ← 200 µl → (1–20) Mischen | | | | | | | | | | | | | | | | | | | |
| Anti-Progesteron-Serum (0-4°C) | | | | ← 200 µl → (3–20) Mischen, Inkubieren 3 Stunden bei 0 - 4°C | | | | | | | | | | | | | | | | | |
| Aktivkohle-Suspension (0-4°C) | | | | ← 500 µl → (3–20) Mischen, Stehenlassen 10 Minuten bei 0-4°C, Zentrifugieren Dekantieren des Überstandes, Messen der Aktivkohle | | | | | | | | | | | | | | | | | |

6. 200 µl des auf 0-4°C gekühlten Anti-Progesteron-Serum werden dann sofort in jedes Reagenzröhrchen (Ausnahme Gesamtaktivität) dispensiert. Alle Röhrchen werden gemixt und dann mit Stopfen oder Parafilm verschlossen.

7. Für die Inkubation bei 0-4°C werden 2-5 h, bevorzugt 3 h angesetzt. Über-Nacht-Inkubation sollte eine Ausnahme sein, da der Assay so an Empfindlichkeit verliert.

8. Die Aktivkohle-Suspension wird sorgfältig auf einem Magnetrührer in einem Eiswasserbad (0-4°C) gerührt und in jedes Reagenzrörchen (Ausnahme Gesamtaktivität) 500 µl dispensiert (Compu-Pet oder Mikropipetten, deren Spitzenöffnungen durch Abschneiden vergrößert sind).

Die Suspension des Aktivkohle-Reagenz muß während des Arbeitsvorganges erhalben bleiben.

9. Alle Röhrchen werden sorgfältig gemixt und 10 min (5-15 min) bei 0-4°C stehengelassen.

10. Die Aktivkohle wird bei 1000 g (750-1500 g) 15 min bei 0-25°C abzentrifugiert und dann der Überstand sofort dekantiert. Die Röhrchen werden, während sie noch umgekehrt sind, 1 min auf Filterpapier-Unterlagen gestülpt und abgedrückt, damit die Restflüssigkeit aufgesaugt wird.

11. Die Aktivkohle wird dann direkt im Gammaprobenwechsler 60 sec gemessen. 20.000 - 40.000 lpm sollten erhalten werden in den Röhrchen "Gesamtaktivität", jedoch ist das vom Alter des Bestecks und dem Wirkungsgrad des Meßgerätes abhängig.

Auswertung der Ergebnisse

Eine typische Standardkurve für die Bestimmung von Progesteron im Serum ist in Fig. 1 dargestellt. Es ist unumgänglich, in jedem Fall von Patientenprobenmessungen eine neue Standardkurve zu erstellen.

Alle gemessenen Daten werden registriert; sie repräsentieren das nicht an Antikörper gebundene Progesteron nach Gleichgewichtsreaktion Progesteron/Antikörper.

Die Impulse pro Minute der Standards $S_0$-$S_5$ werden gegen die zugehörige Progesteron-Konzentration in ein entsprechend vorbereitetes Millimeterpapier eingetragen. Durch die Punkte wird die "ideale" Kurve gezeichnet, wobei stark abweichende Einzelwerte unberücksichtigt bleiben.

Aus den beiden Meßwerten des Testserums und der Patientenserum-Proben werden die Mittelwerte gebildet und hierfür aus der Standardkurve der gesuchten Progesterongehalt pro ml Serum abgelesen.

Es ist für die Auswertung außerdem vorteilhaft, die pro Minute gemessenen Impulse jeweils von der Gesamtaktivität zu subtrahieren und die %-Bindung an Antikörper der Standards $S_1$-$S_5$ bezogen auf die Bindung für Standard $S_0$ (% von $S_0$ = 100) als Ordinate gegen die Progesteron-Konzentration in semilogarithmisches Papier auf der Abszisse einzutragen, und aus diesen Punkten die Standardkurve zu konstruieren.

Beispiel 2

Bestimmung von Progesteron in Milch

Ein Besteck zur radioimmunologischen Progesteron-Bestimmung in Milch besteht aus den folgenden Reagenzien:

1) $^{125}$J-Progesteron, 1 ng $P_4$, 4 µCi $^{125}I$
Lyophilisat (1 Fläschchen)

2) Kaninchen-Anti-Progesteron-Serum
Lyophilisat (1 Fläschchen)

3) Progesteron-Standards, $S_0$ - $S_6$
Konzentration im Bereich von 0 - 40 ng Progesteron/ml
Lyophilisate (7 Fläschchen)

4) Testprobe, Progesteron-Konzentration deklariert
Lyophilisat (1 Fläschchen)

5) Adsorptionsreagenz
2 Tabletten Aktivkohle (1 Fläschchen)

1.Vorbereitung der Reagenzien und Hifsmittel

Tabelle 2: Rekonstitution der Besteck-Reagenzien

| Reagenz | lösen in dest. $H_2O$ |
|---|---|
| Standard $S_0$ - $S_6$ | 500 µl |
| Testprobe | 500 µl |
| $^{125}$J-Progesteron | 20 ml |
| Anti-Progesteron-Serum | 20 ml |
| Aktivkohle-Tabletten | suspendiert in 50 ml |

1.1 Die Fläschchen mit Standards und Testprobe werden kurz aufgestoßen, um eventuell am Stopfen haftende Partikel abzulösen. Dann erst werden die Abreißkappen entfernt, die Gummistopfen vorsichtig gelüftet und jeweils 500 µl destilliertes Wasser einpipettiert. Danach werden die Stopfen wieder eingedrückt. Bis zum vollständigen Lösen der Lyophilisate

läßt man die Fläschchen 10 min. stehen und mixt die Lösungen anschließend kurz auf dem Vortex.

1.2 Vom $^{125}$J-Progesteron und Anti-Progesteron-Serum werden die Abreißkappen und die Gummistopfen sorgfältig entfernt und letztere umgekehrt auf die Arbeitsplatte gelegt. Sowohl das $^{125}$J-Progesteron als auch das Anti-Progesteron-Serum wird in 20 ml destilliertem Wasser gelöst, die entsprechenden Stopfen werden wieder aufgesetzt und die Flaschen ca. 10 min. auf dem Horizontalschüttler bei ca. 200 Upm bewegt oder durch kreisende Bewegungen und/oder Rollen der Fläschchen zwischen den Händen der Lösungsvorgang unterstützt.

1.3 Die Aktivkohle-Tabletten werden in ein 100 ml-Becherglas gegeben und in 50 ml destilliertem Wasser mit Hilfe eines Magnetrührers suspendiert.

1.4 Eine genügende Anzahl von Inkubationsröhrchen (3-5 ml) werden wie in Tabelle 2 angegeben, numeriert und in die Racks gesetzt.

1.5 Ein Eiswasserbad oder Kühltablett wird vorbereitet und alle Reagenzien und die Racks mit Inkubationsröhrchen mindestens 30 min. zur Vorkühlung eingesetzt.

2. Milchproben

Die Milchproben stehen bei + 6°C im Kühlschrank bereit.

3. Durchführung der Bestimmung

(im Eiswasserbad oder auf dem Kühltablett)

3.1 Standards auf dem Vortex mixen und in 20 µl-Aliquots in die dafür vorbereiteten Röhrchen Nr. 3 bis 16 pipettieren.

3.2 Testprobe auf dem Vortex mixen und in 20 µl-Aliquots in die dafür vorbereiteten Röhrchen Nr. 17 und 18 pipettieren.

3.3 Milchproben kräftig mit der Hand schütteln und in 20 µl Aliquots in die dafür vorbereiteten Röhrchen Nr. 19, 20 etc. pipettieren.

3.4 $^{125}$J-Progesteronlösung kurz schütteln und in jedes Röhrchen 200 µl Aliquots dispensieren.

3.5 Gesamtaktivitäts-Röhrchen Nr. 1 und 2 zur Kennzeichnung durch Stopfen verschließen, alle Röhrchen (Ausnahme Gesamtaktivität) auf dem Vortex mixen.

3.6 Anti-Progesteron-Serum kurz schütteln und in jedes Röhrchen 200 µl-Aliquots dispensieren (Ausnahme Gesamtaktivität).

3.7 Alle Röhrchen auf dem Vortex mixen (Ausnahme Gesamtaktivität).

3.8 Röhrhcen in den Racks mit Parafilm$^{(R)}$ abdecken und 4 (3-5) Stunden bei 4°C im Eiswasserbad oder auf dem Kühltablett inkubieren.

Das Eiswasserbad muß über die gesamte Inkubationszeit genügend Eis enthalten, um die Temperatur von 4°C zu gewährleisten. Es hat sich bewährt, wenn Platz vorhanden, das Eiswasserbad während der Inkubationszeit in den Kühlschrank zu stellen.

3.9 Die Aktivkohle-Suspension wird sorgfältig in einer Schale mit Eis und Wasser 10 min. auf einem Magnetrührer gerührt, und unter sorgfältigem ununterbrochenem Rühren werden 500 µl-Aliquots in jedes Inkubationsröhrchen dispensiert. Die Racks verbleiben während dieser Zeit im Eiswasserbad oder auf dem Kühltablett.

3.10 Alle Röhrchen auf dem Vortex mixen, danach 10 Minuten im Eiswasserbad oder auf dem Kühltablett stehen lassen.

3.11 Die Aktivkohle wird dann bei 1.500 g 15 min. bei 4°C zentrifugiert und der Überstand danach sofort dekantiert, und zwar werden die Racks am besten insgesamt umgekehrt und anschließend solange noch umgekehrt auf einer saugfähigen Unterlage kurz abgedrückt, um noch am Röhrchen haftende Flüssigkeit zu entfernen. Dann dreht man das Gestell sofort wieder, um ein Abrutschen der Aktivkohle zu vermeiden.

3.12 Die Aktivkohle wird im Gammaszintillationszähler 1 min. gemessen (Gesamtaktivität 45.000 - 30.000 Ipm).

4. Auswertung

Eine für Milchprogesteron typische Standardkurve sind in Fig. 2 dargestellt.

4.1 Von allen Einzelansätzen der Standards $S_0$ - $S_6$ wird die in Impulsen pro Minute (Ipm) gemessene Radioaktivität gegen die zugehörige Progesteronkonzentration (Konzentrationsangabe auf dem Flaschenetikett!) auf ein vorbereitetes Millimeterpapier eingetragen . Durch die Punkte wird dann die ideale Kurve gezeichnet, wobei stark abweichende Zählraten unberücksichtigt bleiben.

4.2 Es ist für die Auswertung ebenso geeignet, die pro Minute gemessenen Impulse von der Gesamtaktivität abzuziehen und die %-Bindung der Standards $S_1$ - $S_6$ auf den Standard $S_0$ (% von $S_0$ = 100) als

Ordinatenwert gegen die Progesteronkonzentrationen auf der Abszisse auf semilogarithmisches Papier einzutragen und durch diese Punkte die ideale Kurve zu zeichnen.

Anmerkung:

Die Progesteronkonzentration ist in ng/ml anzugeben. Um SI-Einheiten zu erhalten, gilt folgende Umrechnung:

nmol Progesteron/ml = 0,314 ng Progesteron/ml

Figur 4 zeigt den typischen Verlauf der Progesteron-Konzentration in Kuhmilch während eines Zyklus. Die gestrichelten Kurven zeigen die höchsten und die tiefsten beobachteten Werte, die durchgezogene Kurve gibt die Durchschnittskonzentration an.

Tabelle 2:

| | Gesamt-aktivität | | Standards (µl) | | | | | | | | | | | | | | Test-probe (µl) | | Milchproben (µl) | | | |
|---|---|---|---|---|---|---|---|---|---|---|---|---|---|---|---|---|---|---|---|---|---|---|
| Nr. der Teströhrchen | 1 | 2 | 3 | 4 | 5 | 6 | 7 | 8 | 9 | 10 | 11 | 12 | 13 | 14 | 15 | 16 | 17 | 18 | 19 | 20 | 21 | 22 |
| Standards $P_4$-Konzentration deklariert | | | | | | | | | | | | | | | | | | | | | | |
| $S_o$ | | | 20 | 20 | | | | | | | | | | | | | | | | | | |
| $S_1$ | | | | | 20 | 20 | | | | | | | | | | | | | | | | |
| $S_2$ | | | | | | | 20 | 20 | | | | | | | | | | | | | | |
| $S_3$ | | | | | | | | | 20 | 20 | | | | | | | | | | | | |
| $S_4$ | | | | | | | | | | | 20 | 20 | | | | | | | | | | |
| $S_5$ | | | | | | | | | | | | | 20 | 20 | | | | | | | | |
| $S_6$ | | | | | | | | | | | | | | | 20 | 20 | | | | | | |
| Testprobe $P_4$-Konzentr. deklariert | | | | | | | | | | | | | | | | | 20 | 20 | | | | |
| Milchprobe | | | | | | | | | | | | | | | | | | | 20 | 20 | | |
| Milchprobe | | | | | | | | | | | | | | | | | | | | | 20 | 20 |
| $^{125}$I-Progesteron | ← 200 µl → Mixen | | | | | | | | | | | | | | | | | | | | | |
| Anti-Progesteron-Serum | | | ← 200 µl → Mixen, Inkubieren 4 (3 - 5) h bei 4 °C im Eiswasserbad | | | | | | | | | | | | | | | | | | | |
| Aktivkohle-Suspension | | | ← 500 µl → Mixen, Inkubieren 10 Minuten bei 4 °C im Eiswasserbad, Zentrifugieren (> 1.500 g, 15 min. bei 4 °C) Dekantieren des Überstandes, Messen der Aktivkohle | | | | | | | | | | | | | | | | | | | |

Beispiel 3

Untersuchung der Ovulation

Die radioimmunologische Progesteronbestimmung wird wie in Beispiel 1 beschrieben an einer Reihe von Plasmaproben durchgeführt, die von 11 Frauen während des Geschlechtszyklus erhalten wurden. Ebenfalls wird auch der Spiegel von luteinisierendem Hormon bestimmt.

Die bei 10 von den Frauen erhaltenen Ergebnisse, verbunden über die verschiedenen Zykluslängen, sind in der beigefügten Figur 3 angegeben. Der Peak des luteinisierenden Hormons ist als senkrechter Strich am Tag 0 angegeben. Der Anstieg des Progesteronspiegels nach dem Tag 0 zeigt, daß Ovulation stattgefunden hat, und der sich ergebende Abfall im Progesteronspiegel zeigt, daß keine Empfängnis eingetreten ist.

Die bei der radioimmunologischen Bestimmung an den Proben von der elften Frau erhaltenen Ergebnisse waren weitgehend linear und weisen daraufhin, daß keine Ovulation eingetreten war.

Diese Person wurde anschließend mit Noräthisteron (5 mg dreimal täglich)behandelt, um den Zyklus zu regulieren.

Die Ergebnisse bestätigen, daß die erfindungsgemäße radioimmunologische Bestimmung zur Untersuchung von Unfruchtbarkeit verwendbar ist, soweit diese durch mangelnde Ovulation verursacht ist.

Patentansprüche:

1. Radioimmunobesteck zur Bestimmung von Progesteron, dadurch gekennzeichnet, daß es als Reagenzien

   a) ein Progesteronderivat, das in 3-Stellung über eine Brücke mit einem Rest substituiert ist, der ein oder mehrere Atome eines Radiojodisotopes trägt;

   b) gegen ein Progesteron/Proteinkonjugat erzeugte Antikörper, wobei das Protein über eine Brückengruppe in 11-Stellung an das Progesteron gebunden ist;

   c) Progesteronstandards und

   d) ein Trennmittel enthält.

2. Radioimmunobesteck nach Anspruch 1, dadurch gekennzeichnet, daß mindestens ein Reagenz in einem sauren Puffer vorliegt.

3. Verfahren zur radioimmunologischen Bestimmung von Progesteron, dadurch gekennzeichnet, daß man im sauren Medium eine Flüssigkeitsprobe mit einer bekannten Menge von gegen ein Progesteron /Proteinkonjugat erzeugten Antikörper, wobei das Protein über eine Brückengruppe in 11-Stellung an das Progesteron gebunden ist, und mit einer bekannten Menge eines Progesteronderivates, das in 3-Stellung über eine Brücke mit einem Rest substituiert ist, der ein oder mehrere radioaktive Jodisotope trägt, inkubiert, das in dem entstandenen Antikörper/Antigenkomplex gebundene Steroid vom freien Steroid abtrennt und deren Radioaktivität oder die des Antigen/Antikörperkomplexes mißt.

4. Verwendung des Radioimmunbesteckes gemäß Anspruch 1 zur Bestimmung von Progesteron im Serum.

5. Verwendung des Radioimmunbesteckes gemäß Anspruch 1 zur Bestimmung von Progesteron in der Milch.

6. Verwendung des Radioimmunobesteckes gemäß Anspruch 1, zur Feststellung der Schwangerschaft und/oder Ovarialfunktion an Mensch oder Kuh.

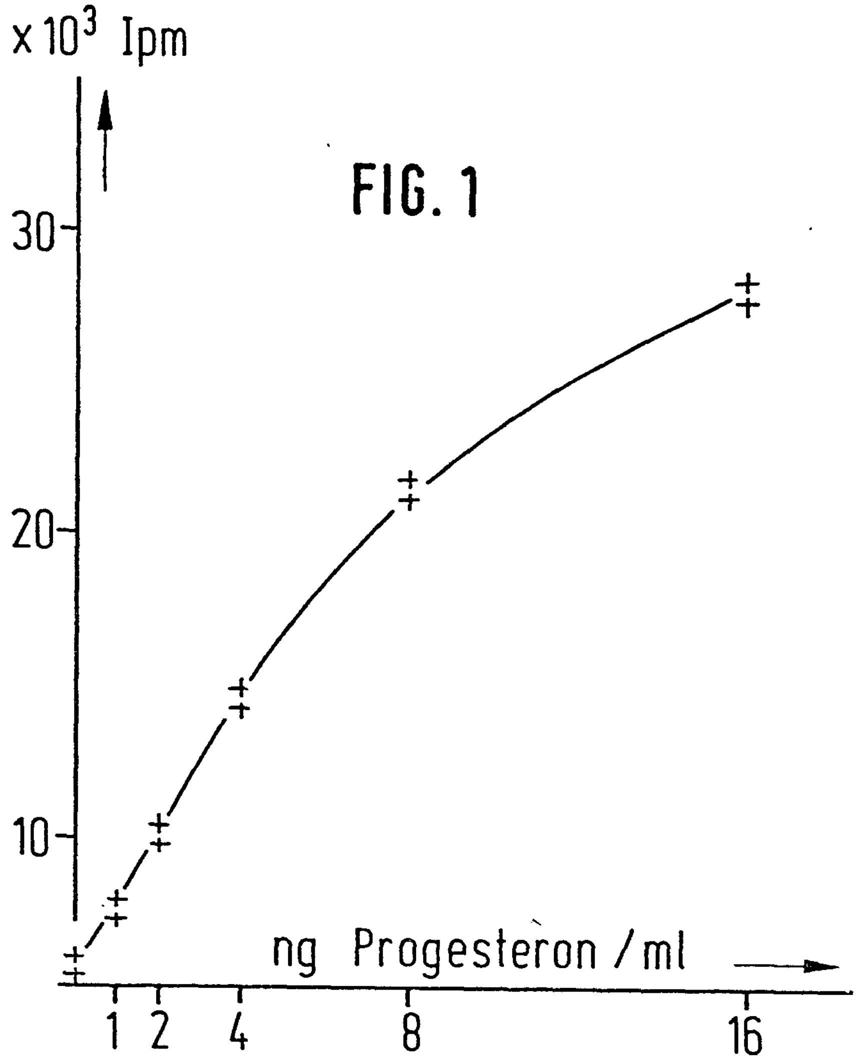


FIG. 1

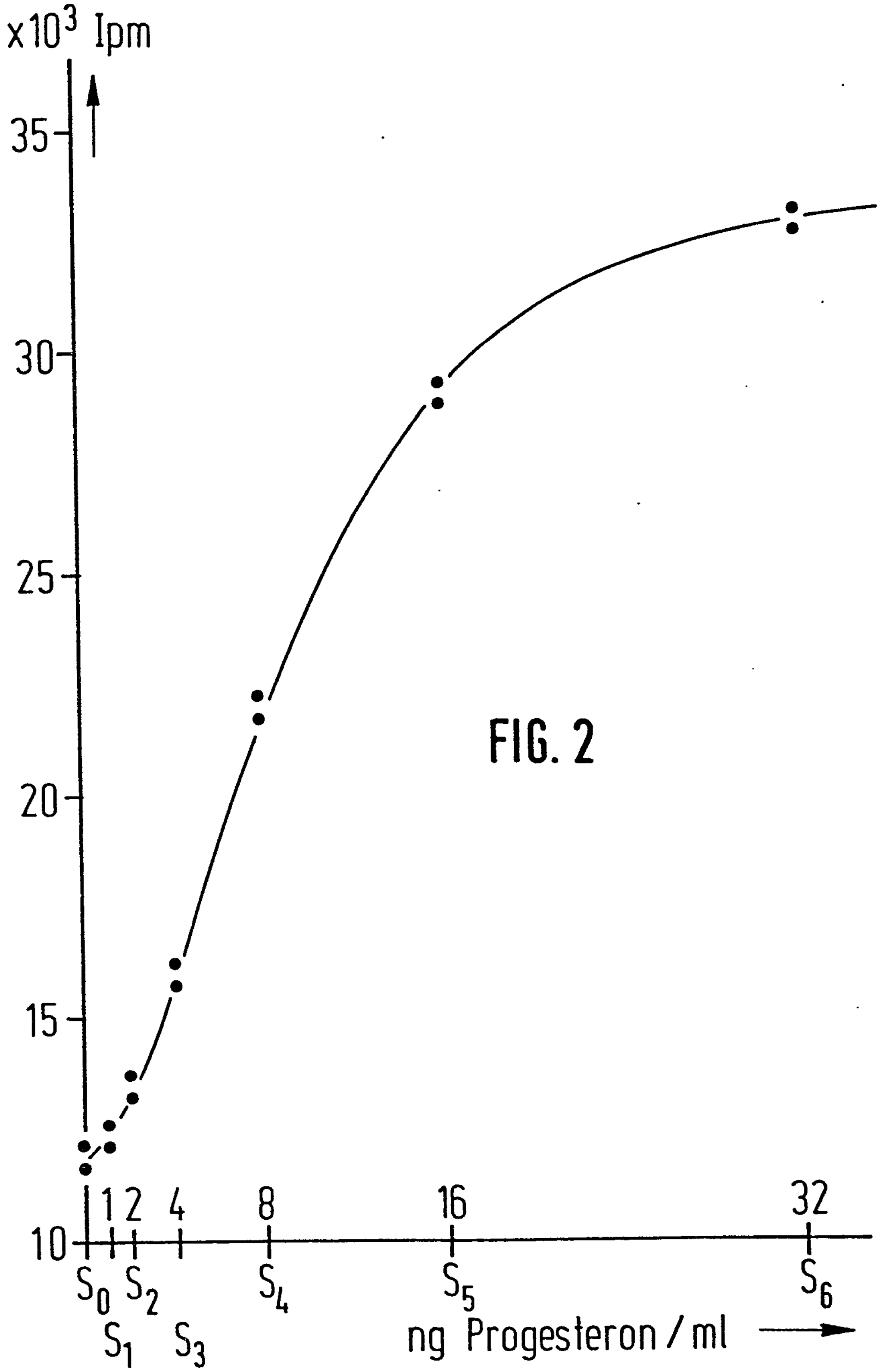
x10³ Ipm
35
30
25
20
15
10
1
2
4
8
16
32
S0
S1
S2
S3
S4
S5
S6
ng Progesteron / ml

FIG. 2

FIG. 3

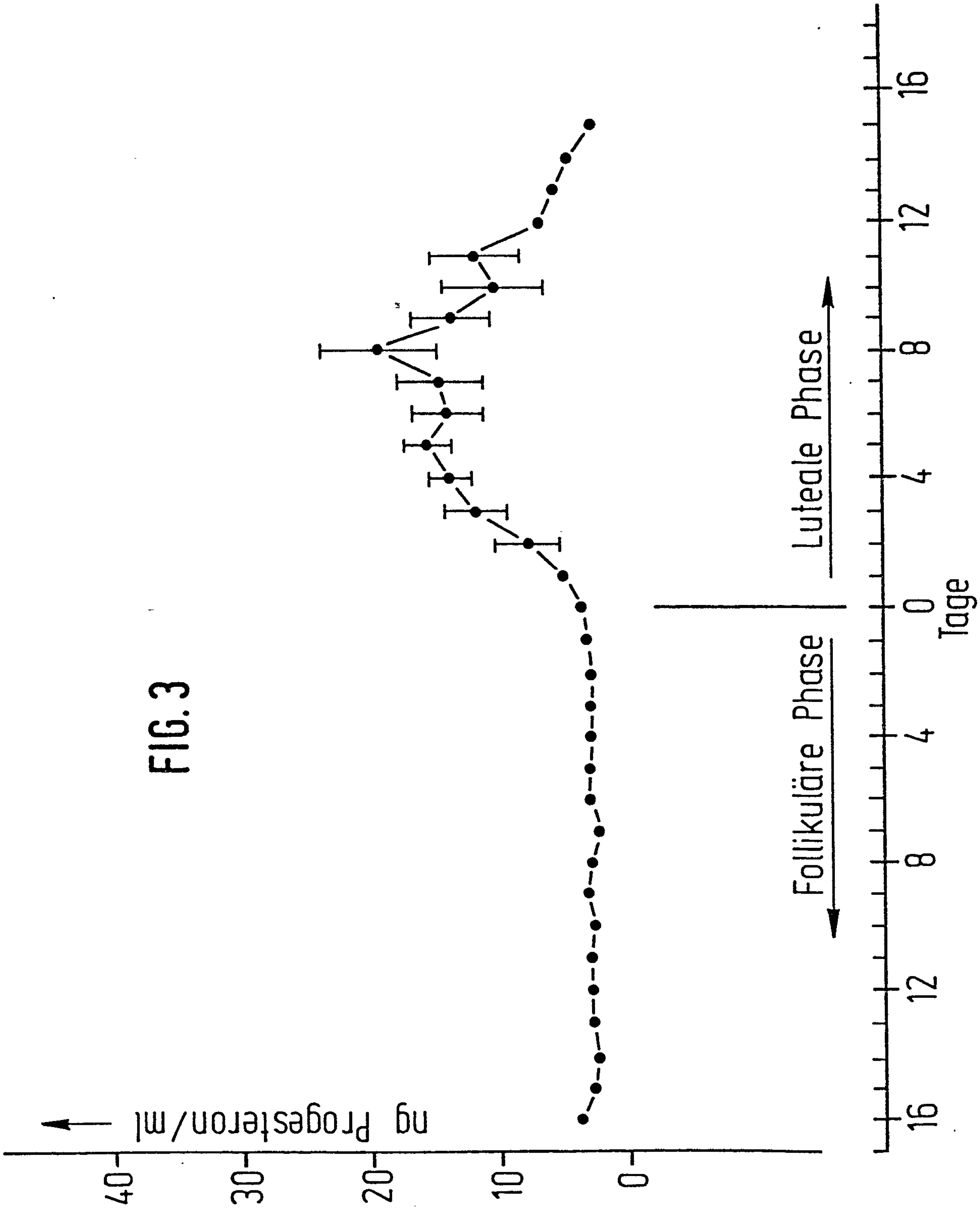
ng Progesteron/ml
40
30
20
10
0
16
12
8
4
0
4
8
12
16
Tage
Follikuläre Phase
Luteale Phase

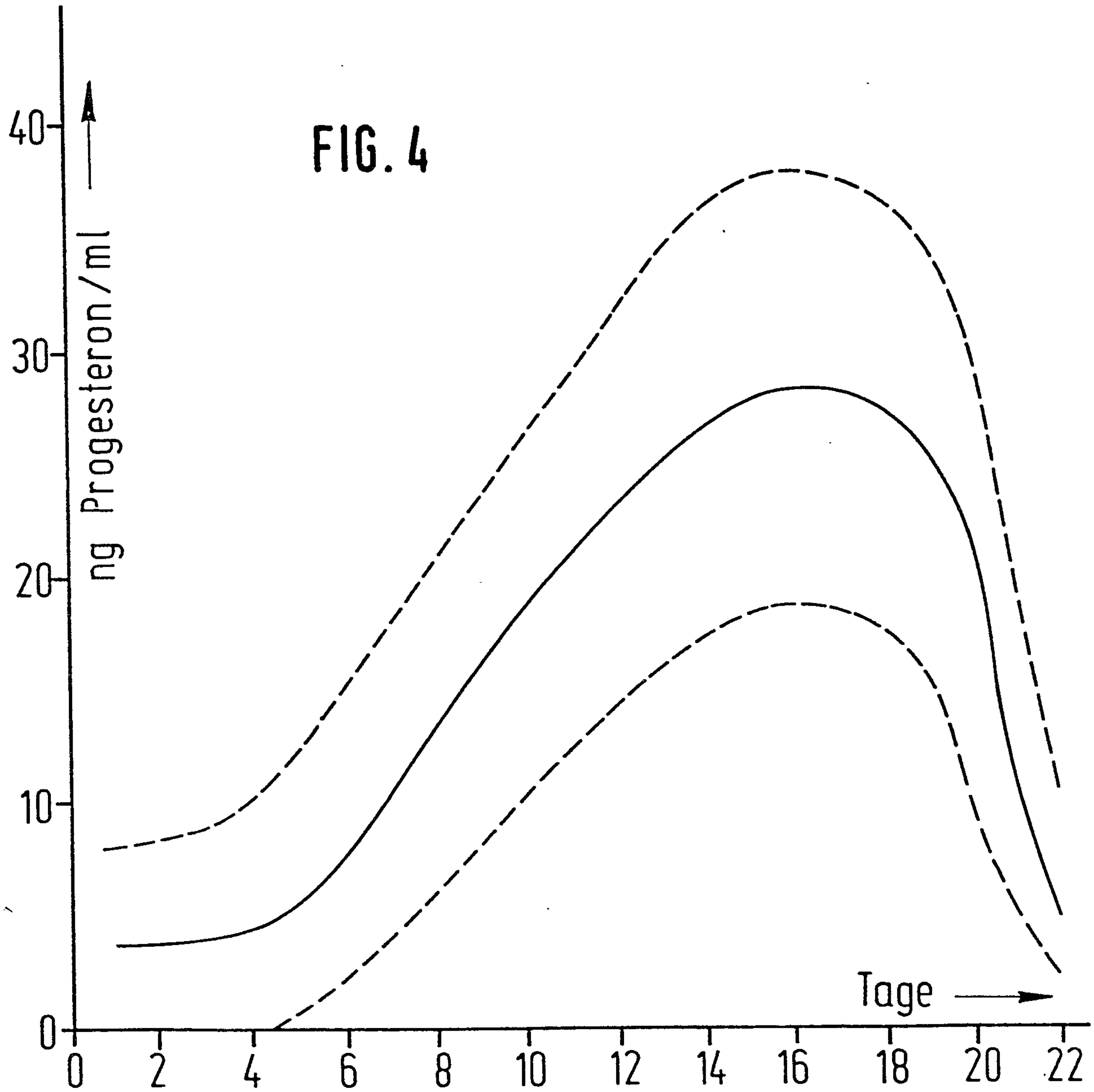


FIG. 4